# EUROPÄISCHE PATENTSCHRIFT

(11) **EP 1 436 300 B1**
(45) Veröffentlichungstag und Bekanntmachung des Hinweises auf die Patenterteilung: **15.10.2008**
(21) Anmeldenummer: 02782812.8
(22) Anmeldetag: 02.10.2002
(51) Int. Cl.: C07F 1/02

(54) **VERFAHREN ZUR METALLIERUNG VON AROMATEN DURCH LITHIUMORGANISCHE VERBINDUNGEN**
METHOD FOR PRODUCING, VIA ORGANOMETALLIC COMPOUNDS, ORGANIC INTERMEDIATE PRODUCTS
PROCEDE DE PRODUCTION, VIA DES ORGANOMETALLIQUES, DE PRODUITS INTERMEDIAIRES ORGANIQUES

(30) Priorität: 12.10.2001 DE 10150615
(43) Veröffentlichungstag der Anmeldung: 14.07.2004
(73) Patentinhaber: Archimica GmbH, 65929 Frankfurt am Main (DE)
(72) Erfinder: MEUDT, Andreas, 65719 Hofheim (DE)
(74) Vertreter: Schweitzer, Klaus
(86) Internationale Anmeldenummer: PCT/EP2002/011042
(87) Internationale Veröffentlichungsnummer: WO 2003/033503

(56) Entgegenhaltungen:
- WO-A-00/64905
- GIMLAN, H., MOORE, F.W. AND OGDEN, B.: J. AMER. CHEM. SOC., Bd. 63, 1941, Seiten 2479-2482, XP002228260
- BARTLETT, P.D., SWAIN, C.G. AND WOODWARD, R.B.: J. AMER. CHEM. SOC., Bd. 63, 1941, Seiten 3229-3230, XP002228261
- JOHNSON, O.H., EBERGALL, W.H.: J. AMER. CHEM. SOC., Bd. 71, 1949, Seiten 1720-1722, XP002228262
- TARBELL, D.S., WEISS, M.: J. AMER. CHEM. SOC., Bd. 61, 1939, Seiten 1203-1205, XP002228263
- SCHLOSSER, M.: "Organometallics in Synthesis, A Manual" 2002 , JOHN WILEY & SONS, LTD , CHICHESTER, ENGLAND XP002228264 Seite 223 -Seite 247 Seite 341 -Seite 347

## Beschreibung

Die Erfindung betrifft ein Verfahren zur Herstellung von organischen Verbindungen durch Erzeugung von Aryllithiumverbindungen und deren Umsetzung mit geeigneten Elektrophilen, wobei zunächst durch Umsetzung von Halogenverbindungen mit Lithiummetall eine Lithiumverbindung generiert wird, das anschließend zur Deprotonierung von aromatischen Verbindungen unter Bildung des gewünschten Lithiumaromaten umgesetzt wird, wobei dieser abschließend gegebenenfalls mit einem entsprechenden Elektrophil zur Reaktion gebracht wird (Gleichung I),

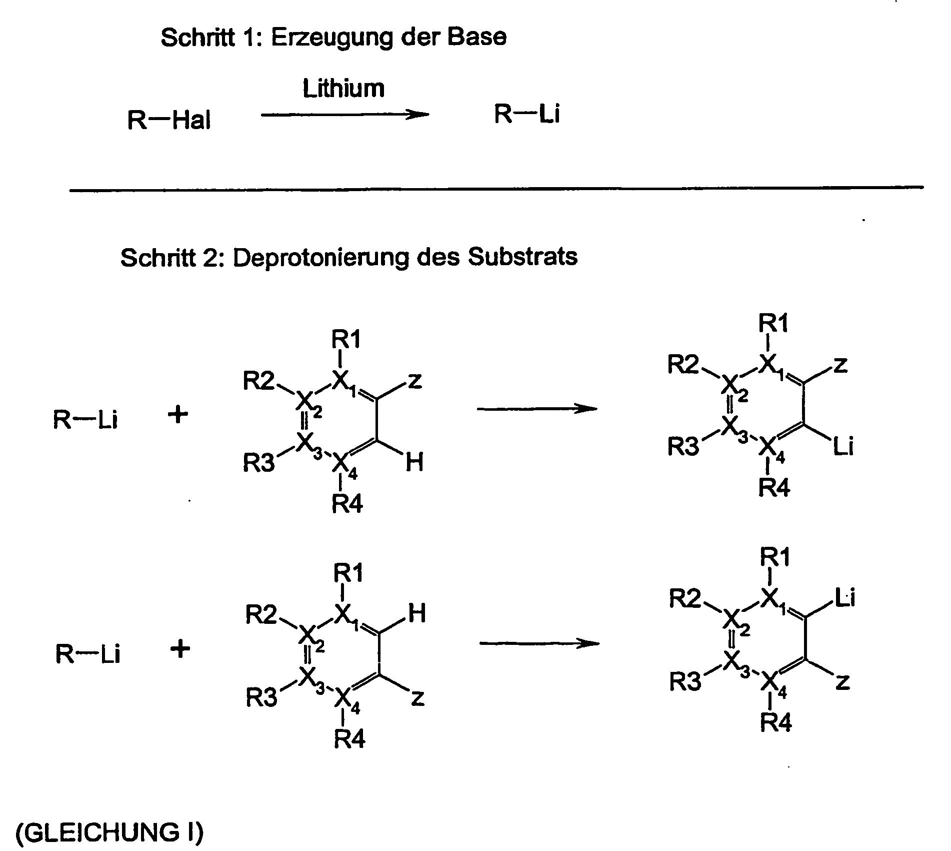

Der Aufschwung der metallorganischen Chemie, insbesondere derjenige des Elementes Lithium, in der Herstellung von Verbindungen für die pharmazeutische und agrochemische Industrie sowie für zahlreiche weitere Anwendungen ist in den vergangenen Jahren beinahe exponentiell verlaufen, wenn man die Anzahl der Anwendungen bzw. die Menge entsprechend hergestellter Produkte gegen eine Zeitachse aufträgt. Wesentliche Gründe hierfür sind die immer komplexer werdenden Strukturen der benötigten Feinchemikalien für die Bereiche Pharma und Agro einerseits sowie das fast unbegrenzte Synthesepotential der Lithiumorganyle für den Aufbau komplexer organischer Strukturen andererseits.

Beinahe jede lithiumorganische Verbindung lässt sich über das moderne Arsenal der metallorganischen Chemie leicht erzeugen und mit fast jedem Elektrophil zum jeweils gewünschten Produkt umsetzen. Dabei werden die meisten Lithiumorganyle auf einem der folgenden Wege generiert:
(1) Zweifellos der wichtigste Weg ist der Halogen-Metall-Austausch, bei dem meist Bromaromaten mit n-Butyllithium bei tiefen Temperaturen umgesetzt werden
(2) Durch Umsetzung von Bromaromaten mit Lithiummetall lassen sich ebenfalls sehr viele Li-Organyle herstellen
(3) Sehr bedeutsam ist des weiteren die Deprotonierung von organischen Verbindungen mit Lithiumalkylen (z. B. BuLi) oder Lithiumamiden (z. B. LDA oder LiNSi)

Daraus folgt schon, dass für den größten Teil dieser Chemie der Einsatz von käuflichen Alkyllithium-Verbindungen erforderlich ist, wobei hier meistens n-BuLi eingesetzt wird. Die Synthese von n-BuLi und verwandten Lithiumaliphaten ist technisch aufwendig und erfordert sehr viel Know-How, wodurch n-Butyllithium, s-Butyllithium, tert.-Butyllithium und ähnliche Moleküle nur - an industriellen Maßstäben gemessen - sehr teuer angeboten werden. Dies ist der wichtigste, aber bei weitem nicht der einzige Nachteil dieses ansonsten sehr vorteilhaft und breit einsetzbaren Reagenzes.

Aufgrund der extremen Empfindlichkeit und in konzentrierten Lösungen pyrophorer Natur solcher Lithiumaliphaten müssen bei den in der industriellen Großproduktion angestrebten Größenordnungen (Jahresproduktionsmengen zwischen 5 und 500 Tonnen) sehr aufwendige logistische Systeme für Transport, Einspeisung in die Dosiervorlage und Dosierung aufgebaut werden, die hohen Investitionen in das Anlagevermögen entsprechen.

Des weiteren entstehen bei den Umsetzungen von n-, s- und tert.-Butyilithium entweder Butane (Deprotonierungen), Butylhalogenide (Halogen-Metallaustausch, 1 Äquivalent BuLi) oder Buten und Butan (Halogen-Metall-Austausch), die bei Raumtemperatur gasförmig sind und bei den erforderlichen hydrolytischen Aufarbeitungen den Reaktionsmischungen entweichen. Dadurch werden zusätzlich auch noch aufwendige Abgasaufreinigungen oder entsprechende Verbrennungsvorrichtungen erforderlich, um den strengen gesetzlichen Immissionsvorschriften zu genügen. Als Ausweg bieten die spezialisierten Unternehmen Alternativen wie n-Hexyllithium an, die zwar keine Butane entstehen lassen, dafür aber nochmals deutlich teurer sind als Butyllithium.

Ein weiterer Nachteil ist das Anfallen komplexer Lösungsmittelgemische nach der Aufarbeitung. Aufgrund der hohen Reaktivität von Alkyllithium-Verbindungen gegenüber Ethern, die fast immer Lösungsmittel für die Folgeumsetzungen sind, können Alkyllithium-Verbindungen meist nicht in diesen Lösungsmitteln angeboten werden. Die Hersteller bieten zwar eine breite Palette von Alkyllithium-Verbindungen verschiedenster Konzentrationen in verschiedensten Kohlenwasserstoffen an, allerdings laufen beispielsweise Halogen-Metall-Austausche in reinen Kohlenwasserstoffen nicht ab, so dass man zwangsläufig in Gemischen aus Ethern und Kohlenwasserstoffen arbeiten muss. Daher erhält man nach Hydrolyse wasserhaltige Gemische aus Ethern und Kohlenwasserstoffen, deren Auftrennung aufwendig ist und in vielen Fällen gar nicht ökonomisch durchführbar ist. Für eine industrielle Großproduktion ist allerdings die Rückführung der verwendeten Lösungsmittel unabdingbare Voraussetzung.

Aus den genannten Gründen wäre es daher sehr wünschenswert, ein Verfahren zu haben, bei dem die zur Deprotonierung einzusetzende Alkyllithiumverbindung ausgehend von den billigen Rohstoffen Halogenalkan und Lithiummetall in einem Ether erzeugt und dabei gleichzeitig oder anschließend mit dem zu deprotonierenden Substrat umgesetzt wird, da durch diese Vorgehensweise alle oben genannten Nachteile der "klassischen" Erzeugung von Lithiumaromaten umgangen werden könnten.

Die vorliegende Erfindung löst alle diese Aufgaben und betrifft ein Verfahren zur Herstellung von Aryllithiumverbindungen der Formeln (IV) und (VI), sowie gegebenenfalls weiterer Umsetzung dieser Verbindungen mit geeigneten Elektrophilen, wobei zunächst durch Umsetzung von Halogenverbindungen (I) mit Lithiummetall eine Lithiumverbindung (11) generiert wird, und diese mit aromatischen Verbindungen der Formeln (III) und/oder (V) unter Deprotonierung und Bildung der gewünschten Lithiumaromaten (IV) und/oder (VI) umgesetzt wird, wobei die Reaktion als Eintopfreaktion durchgeführt wird und die Verbindungen (I), (III) und/oder (V) gleichzeitig oder als Mischung zu der Reaktionslösung zudosiert werden und die zuzusetzende Lithiummenge pro Mol umzusetzendes Halogen im Bereich von 1,95 bis 2,5 mol liegt (Gleichung I). 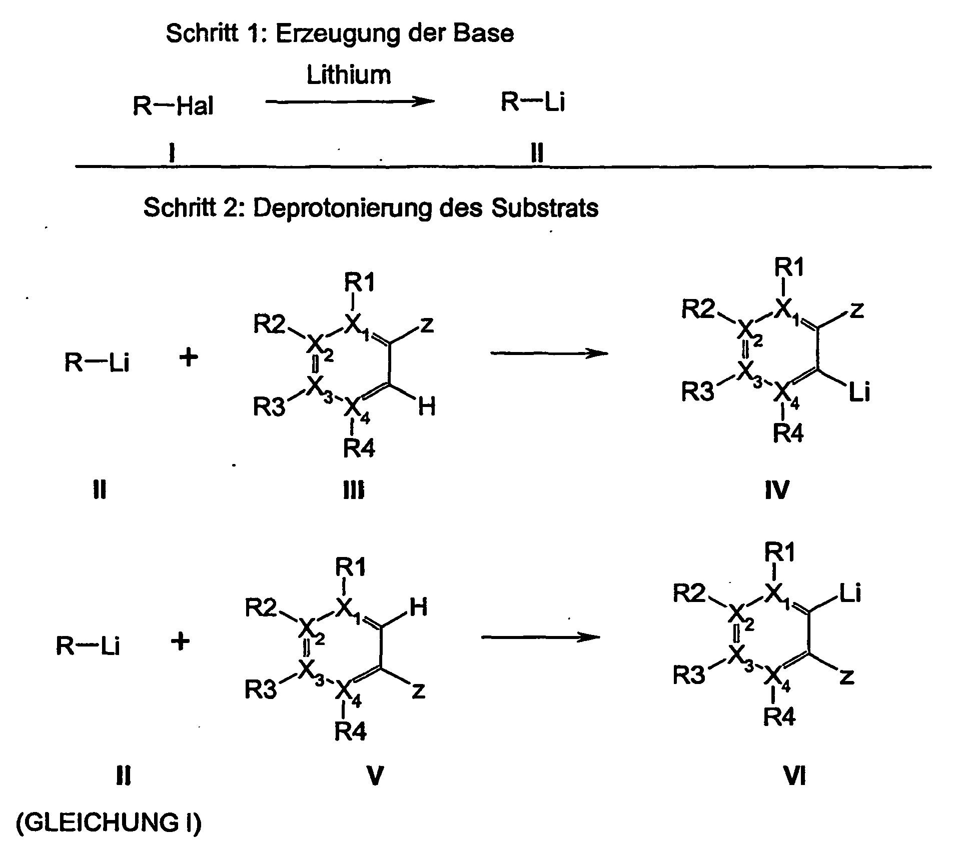 worin R für Methyl, primäre, sekundäre oder tertiäre Alkylreste mit 2 bis 12 C-Atomen, mit einem Rest aus der Gruppe {Phenyl, substituiertes Phenyl, Aryl, Heteroaryl, Alkoxy, Dialkylamino, Alkylthio} substituiertes Alkyl, substituiertes oder unsubstituiertes Cycloalkyl mit 3 bis 8 C-Atomen,
Hal = Fluor, Chlor, Brom oder Iod bedeuten,
X₁-₄ stehen unabhängig voneinander für Kohlenstoff, die Gruppierung X₁₋₄ R₁₋₄ kann Stickstoff bedeuten, oder zwei benachbarte Reste X₁₋₄ R₁₋₄ können gemeinsam für O (Furane), S (Thiophene), NH oder NR' stehen, wobei R' für C₁-C₅-Alkyl, SO₂-Phenyl, SO₂-p-Tolyl oder Benzoyl steht.

Bevorzugte Verbindungen der Formel (III), die nach dem erfindungsgemäßen Verfahren umgesetzt werden können, sind z.B. Benzole, Pyridine, Pyridazine, Pyrimidine, Pyrazine, Furane, Thiophene, N-substituierte Pyrrole, Benzofurane, Indole oder Naphthaline, um nur einige zu nennen.

die Reste R₁₋₄ und der Rest Z stehen für Substituenten aus der Gruppe {Wasserstoff, Methyl, primäre, sekundäre oder tertiäre, cyclische oder acyclische Alkylreste mit 2 bis 12 C-Atomen, substituierte cyclische oder acyclische Alkylgruppen, Alkoxy, Dialkylamino, Alkylamino, Arylamino, Diarylamino, Phenyl, substituiertes Phenyl, Alkylthio, Diarylphosphino, Dialkylphosphino, Dialkyl- oder Diarylaminocarbonyl, Monoalkyl- oder Monoarylaminocarbonyl, CO₂⁻, Hydroxyalkyl, Alkoxyalkyl, Fluor oder Chlor, CN oder Heteroaryl-}, wobei jeweils zwei benachbarte Reste R₁₋₄ zusammen einem aromatischen oder aliphatischen Ring entsprechen können.

Die so hergestellten Lithiumorganyle können mit beliebigen elektrophilen Verbindungen nach Verfahren des Standes der Technik umgesetzt werden. Durch Umsetzung mit Kohlenstoff-Elektrophilen können beispielsweise C,C-Verknüpfungen vorgenommen werden, durch Umsetzung mit Borverbindungen können Boronsäuren hergestellt werden, und durch Umsetzung mit Halogen- oder Alkoxysilanen wird ein effizienter Weg zu Organosilanen erschlossen.

Als Halogenaliphaten können alle verfügbaren oder herstellbaren Fluor-, Chlor-, Brom- oder lodaliphaten eingesetzt werden, da Lithiummetall in etherischen Lösungsmitteln mit allen Halogenaliphaten leicht und in fast allen Fällen mit quantitativen Ausbeuten reagiert. Bevorzugt werden hierbei Chlor- oder Bromaliphaten eingesetzt, da Iodverbindungen oft teuer sind, Fluorverbindungen zur Bildung von LiF führen, das bei späteren wässrigen Aufarbeitungen als HF zu Werkstoffproblemen führen kann. In Spezialfällen können aber auch solche Halogenide vorteilhaft einsetzbar sein.

Gemäß des erfindungsgemäßen Verfahrens werden Bevorzugt solche Alkylhalogenide eingesetzt, die nach der Deprotonierung zu flüssigen Alkanen umgesetzt werden können. Besonders bevorzugt werden Chlor- oder Bromcyclohexan, Benzylchlorid, tert.-Butylchlorid, Chlorhexane oder Chlorheptane eingesetzt.

Die Reaktion wird in einem geeigneten organischen Lösungsmittel durchgeführt, bevorzugt sind etherische Lösungsmittel, beispielsweise Tetrahydrofuran, Dioxan, Diethylether, Di-n-butylether, Diisopropylether oder Anisol, bevorzugt wird THF eingesetzt.

Aufgrund der hohen Reaktivität von Alkyl- und Aryllithiumverbindungen, insbesondere auch gegenüber den als Lösungsmittel eingesetzten Ethern, liegen die bevorzugten Reaktionstemperaturen im Bereich von -100 bis +25°C, besonders bevorzugt sind Temperaturen von -80 bis -25°C.

Ein weiterer Vorteil des erfindungsgemäßen Verfahrens ist, dass bei recht hohen Konzentrationen an lithiumorganischen Verbindungen gearbeitet werden kann. Bevorzugt sind Konzentrationen der aliphatischen bzw. aromatischen Zwischenprodukte der Formel (II) von 5 bis 30 Gew.-%, insbesondere 12 bis 25 Gew.-%.

Gemäß der Erfindung werden Halogenalkan und aromatisches Substrat gleichzeitig oder als Mischung zu Lithiummetall im Ether zudosiert, Bei diesem Eintopfverfahren (gleichzeitige Zudosierung von (I), (III) und/oder (IV) zu Lithium in Ether) bildet sich zunächst der Lithiumaliphat, der dann sofort den Aromaten deprotoniert.

Überraschenderweise haben wir gefunden, dass bei der Ausführungsform als Eintopf in fast allen Fällen deutlich höhere Ausbeuten beobachtet werden, als wenn man zunächst RLi generiert und anschließend erst das aromatische Substrat zugibt.

Das Lithium kann im vorliegenden Verfahren als Dispersion, Pulver, Späne, Sand, Granallen, Stücke, Barren oder in anderer Form eingesetzt werden, wobei die Größe der Lithiumpartikel nicht qualitätsrelevant ist, sondern lediglich die Reaktionszeiten beeinflusst. Daher sind kleinere Partikelgrößen bevorzugt, beispielsweise Granalien, Pulver oder Dispersionen. Die zugesetzte Lithiummenge beträgt je Mol umzusetzenden Halogens 1,95 bis 2,5 mol, bevorzugt 1,98 bis 2,15 Mol.

In allen Fällen können durch Zusatz organischer Redoxsysteme bei der Umsetzung des Li-Metalls in der ersten Stufe, beispielsweise Biphenyl, 4,4'-Di-tert.-butylbiphenyl oder Anthracen, deutliche Steigerungen der Reaktionsgeschwindigkeiten beobachtet werden. Der Zusatz solcher Systeme erwies sich vor allem dann als vorteilhaft, wenn die Lithüerungszeiten ohne diese Katalyse > 12 h waren.

Für die Deprotonierung einsetzbare Aromaten sind zunächst alle Verbindungen, die ausreichend acide sind, um unter den erfindungsgemäßen Bedingungen deprotoniert werden zu können. Hier sind zunächst alle die Aromaten zu nennen, die "ortho-directing substituents" Z haben, also insbesondere Aromaten, die Alkoxy, F, Cl, substituiertes Amino, CN, Heteroaryl. Aminoalkyl, Hydroxyalkyl oder ähnliche Reste tragen. Die Wirkungsweise solcher Reste ist darin begründet, dass diese Substituenten die Koordination des Lithiumions der aliphatischen Base ermöglichen, wodurch das Gegenion R⁻ dann sehr leicht in ortho-Stellung deprotonieren kann.

Des weiteren sind hier alle durch die Kombination mehrerer Effekte stark aciden Heterocyclen wie beispielsweise Furan zu nennen. Hier sind u. a. durch induktiven Effekt des Sauerstoffs sowie durch die sp²-Hybridisierung und die Winkelspannung am α-Kohlenstoff die Protonen ausreichend acide, um die Deprotonierung zu ermöglichen. Entsprechendes gilt für andere Heterocyclen.

In den anderen Fällen, in denen die zu ersetzenden aromatischen Protonen nicht ausreichend acide sind, kann die Deprotonierung dennoch ermöglicht werden, indem Hilfsstoffe zugesetzt werden, die dem Fachmann für derartige Probleme geläufig sind. Besonders bewährt hat sich hierbei Kalium-tert.-butylat, das in Mengen von 0,05 bis 1,2 Äquivalenten schon während der Lithiierung des Halogenaliphaten der Reaktionsmischung zugesetzt wird. Dadurch gelingt selbst die Lithiierung des wenig aciden Benzols (in einigen Fällen entsteht bei derartiger Vorgehensweise teilweise oder auch vollständig der Kaliumaromat, da dies aber keine Auswirkungen auf die Natur der entstehenden Reaktionsprodukte hat, soll dieser Aspekt hier vernachlässigt werden).

Die erfindungsgemäß generierten Lithiumaromaten können nach den dem Fachmann geläufigen Methoden mit elektrophilen Verbindungen umgesetzt werden, wobei insbesondere Kohlenstoff-, Bor- und Silicium-Elektrophile im Hinblick auf die benötigten Zwischenprodukte für die pharmazeutische und agrochemische Industrie von Interesse sind.
Die Umsetzung mit dem Elektrophil kann entweder nach der Erzeugung der lithiierten Verbindung ((IV) und/oder (VI) oder, wie bereits vorher beschrieben, im Eintopfverfahren durch gleichzeitige Zugabe zur Reaktionsmischung erfolgen.

Die Kohlenstoff-Elektrophile stammen insbesondere aus einer der folgenden Kategorien (in Klammern jeweils die Produkte):
Aryl- oder Alkylcyanate (Benzonitrile)
Oxiran, substituierte Oxirane (ArCH₂CH₂OH, ArCR₂CR₂OH) mit R = R¹ (gleich oder verschieden)
Azomethine (ArCR¹₂-NR'H)
Nitroenolate (Oxime)
Immoniumsalze (Aromatische Amine)
Halogenaromat, Aryltriflate, andere Arylsulfonate (Biaryle)
Kohlendioxid (ArCOOH)
Kohlenmonoxid (Ar-CO-CO-Ar)
Aldehyde, Ketone (ArCHR¹-OH, ArCR¹₂-OH;
α,β-ungesättigte Aldehyde/Ketone (ArCH(OH)Vinyl, CR¹(OH)-Vinyl)
Ketene (ARC(=O)CH₃ bei Keten, ArC(=O)-R¹ bei substituierten Ketenen)
Alkali- und Erdalkalisalze von Carbonsäuren (ArCHO bei Formiaten, ArCOCH₃ bei Acetaten, ArR¹CO bei R¹COOMet)
Aliphatische Nitrile (ArCOCH₃ bei Acetonitril, ArR¹CO bei R¹CN)
Aromatische Nitrile (ArCOAr')
Amide (ArCHO bei HCONR₂, ArC(=O)R bei RCONR'₂)
Ester (Ar₂C(OH)R¹) oder
Alkylierungsmittel (Ar-Alkyl).

Als Bor-Elektrophile werden Verbindungen der Formel BW₃ eingesetzt, worin W unabhängig voneinander für gleiche oder verschiedene (C₁-C₆-Alkoxy), Fluor, Chlor, Brom, Iod, N(C₁-C₆-Alkyl)₂ oder S(C₁-C₅-Alkyl) steht, eingesetzt, bevorzugt sind dabei Trialkoxyborane, BF₃*OR₂, BF₃*THF, BCl₃ oder BBr₃, besonders bevorzugt Trialkoxyborane.

Als Silicium-Elektrophile werden Verbindungen der Formel SiW₄ eingesetzt, worin W unabhängig voneinander für gleiche oder verschiedene (C₁-C₆-Alkoxy), Fluor, Chlor, Brom, Iod, N(C₁-C₆-Alkyl)₂ oder S(C₁-C₅-Alkyl) steht, eingesetzt, bevorzugt sind dabei Tetraalkoxysilane, Tetrachlorsilane oder substituierte Alkyl- bzw. Aryl-Halogensilane oder substituierte Alkyl- bzw. Aryl-Alkoxysilane.

Das erfindungsgemäße Verfahren eröffnet eine sehr ökonomische Methode, um die Transformation von aromatischem Wasserstoff in beliebige Reste auf einem sehr wirtschaftlichen Weg zu bewerkstelligen.

Die Aufarbeitungen geschehen im allgemeinen wässrig, wobei entweder Wasser bzw. wässrige Mineralsäuren zudosiert werden oder die Reaktionsmischung auf Wasser bzw. wässrige Mineralsäuren dosiert wird. Zur Erzielung bester Ausbeuten wird hier jeweils der pH-Wert des zu isolierenden Produkts eingestellt, also für gewöhnlich ein leicht saurer, im Falle von Heterocyclen auch leicht alkalischer pH-Wert. Die Reaktionsprodukte werden beispielsweise durch Extraktion und Eindampfen der organischen Phasen gewonnen, alternativ können auch aus der Hydrolysemischung die organischen Lösungsmittel abdestilliert und das dann ausfallende Produkt durch Filtration gewonnen werden.

Die-Reinheiten der Produkte-aus den erfindungsgemäßen Verfahren sind im allgemeinen hoch, für Spezialanwendungen (Pharmavorprodukte) kann allerdings noch ein weiterer Aufreinigungsschritt, beispielsweise durch Umkristallisation unter Zusatz geringer Mengen Aktivkohle, erfordedich werden. Die Ausbeuten an den Reaktionsprodukten betragen zwischen 70 und 99 %, typische Ausbeuten sind insbesondere 85 bis 95 %.

Das erfindungsgemäße Verfahren soll durch die nachfolgenden Beispiele erläutert werden, ohne die Erfindung darauf zu beschränken:

### Beispiel 1

Herstellung von 2,6-Dimethoxyphenylboronsäure aus Resorcindimethylether und Chlorcyclohexan

Eine Mischung aus 20,88 g Chlorcyclohexan (0,176 mol) und 22,1 g Resorcindimethylether (0,16 mol) wird zu einer Suspension von 2,35 g Lithiumgranalien (0,34 mol) in 300 g THF bei -50°C zugetropft, wobei als Dosierzeit 2 Stunden gewählt wurde. Nach einem per GC bestimmten Umsatz des Chlorcyclohexans von > 97 % (insgesamt 9 h) werden bei der gleichen Temperatur 16,6 g Trimethylborat (0,16 mol) in 15 Minuten zugetropft. Nach 30-minütigem Nachrühren bei -50°C wird die Reaktionsmischung auf 120 g Wasser gegeben, der pH mit 37 % HCl auf 6,3 eingestellt und THF und Cyclohexan bei 35°C im Vakuum abdestilliert. Man gibt 25 ml Methylcyclohexan zu der Produktsuspension, saugt das farblose Produkt ab und wäscht einmal mit 25 ml kaltem Methylcyclohexan und einmal mit 25 ml kaltem Wasser nach. Man erhält nach Trocknung 26,5 g 2,6-Dimethoxyphenylboronsäure (0,146 mol, 91 %, Schmelzpunkt 104-107°C) in Form farbloser Kristalle, HPLC-Reinheit > 99 % a/a.

### Beispiel 2

Herstellung von 5-Formylfuran-2-boronsäure aus Furfural-Diethylacetal und Chlorcyclohexan

Eine Mischung aus 20,88 g Chlorcyclohexan (0,176 mol) und 27,2 g Furfural-Diethylacetal (0,16 mol) wird zu einer Suspension von 2,35 g Lithiumgranalien (0,34 mol) in 300 g THF bei -65°C zugetropft, wobei als Dosierzeit 2 Stunden gewählt wurde. Nach einem per GC bestimmten Umsatz des Chlorcyclohexans von > 97 % (insgesamt 10 h) werden bei der gleichen Temperatur 18,3 g Trimethylborat (0,176 mol) in 30 Minuten zugetropft. Nach 30-minütigem Nachrühren bei -65°C wird die Reaktionsmischung auf 120 g Wasser gegeben, der pH mit 37 % HCl auf 6,3 eingestellt und THF und Cyclohexan bei max. 35°C im Vakuum abdestilliert. Anschließend stellt man den pH auf 1,5 ein, rührt bis zur vollständigen Ausfällung des Produkts und filtriert ab. Nach Waschen mit wenig kaltem Wasser und wenig kaltem Aceton erhält man nach Trocknung 17,2 g 5-Formyl-2-furanboronsäure (0,123 mol, 77 %) in Form eines beigefarbenen, feinen Pulvers, HPLC-Reinheit > 99 % a/a.

### Beispiel 3

Herstellung von Salicylsäuremethylether aus Anisol und Chlorcyclohexan

Eine Mischung aus 20,88 g Chlorcyclohexan (0,176 mol) und 17,3 g Anisol (0,16 mol) wird zu einer Suspension von 2,35 g Lithiumgranalien (0,34 mol) in 300 g THF bei -50°C zugetropft, wobei als Dosierzeit wiederum 2 Stunden gewählt wurde. Nach einem per GC bestimmten Umsatz des Chlorcyclohexans von > 97 % (insgesamt 11 h) wird bei der gleichen Temperatur wasserfreies Kohlendioxid eingeleitet, bis die Lösung mit CO2 gesättigt ist. Nach 30-minütigem Nachrühren bei -50°C wird die Reaktionsmischung auf 100 g Wasser gegeben, der pH mit 37 % HCl auf 3,4 eingestellt und die Lösungsmittel bei max. 55°C im Vakuum abdestilliert. Man saugt das farblose Produkt ab und erhält nach Trocknung Salicylsäuremethylether (Ausbeute 79 %) in Form farbloser Kristalle, HPLC-Reinheit > 99 % a/a. Durch Extraktion der Mutterlauge mit Dichlormethan, Trocknen mit Natriumsulfat und Eindampfen kann weiterer Salicylsäuremethylether erhalten werden, Gesamtausbeute 93 %.

### Beispiel 4

Herstellung von 2,6-Difluoracetophenon aus 1,3-Difluorbenzol und Essigsäureanhydrid

Es wird zunächst eine Lösung von tert.-Butyllithium in THF erzeugt, indem 9,25 g tert.-Butylchlorid mit 1,4 g Lithiumgranalien in 100 g THF bei -78°C umgesetzt werden. Nach Erreichen eines Umsatzes von > 97 % (GC a/a) wird 1,3-Difluorbenzol (11,4 g) zugegeben und 30 Minuten bei -78°C, anschließend noch 2 h bei -65°C nachgerührt. Die erhaltene Lösung von 2,6-Difluor-1-lithiobenzol wird in eine auf -5°C gekühlte Lösung von 22 g Essigsäureanhydrid in 35 g THF getropft. Nach der üblichen wässrigen Aufarbeitung erhält man 2,6-Difluoracetophenon in einer Ausbeute von 92 %.

### Beispiel 5

Herstellung von 2-(Thiophenyl)-ethanol aus Thiophen und 1-Chlorheptan

Eine Mischung aus 145 g 1-Chlorheptan (1,1 mol) und 84,0 g Thiophen (1,0 mol) wird in 3 h zu einer Suspension von 14,5 g Lithiumgranalien (2,1 mol) in 500 g THF bei -50 °C getropft. Nach einem per GC bestimmten Umsatz des Chlorheptan von > 97 % (insgesamt 9 h) werden bei der gleichen Temperatur 48 g Ethylenoxid (1,1 mol) eingeleitet. Nach 30-minütigem Nachrühren bei -50°C wird die Reaktionsmischung auf 120 g Wasser gegeben, der pH mit 37 % HCl auf 5,9 eingestellt und die Niedrigsieder bei max. 55°C im Vakuum abdestilliert. Nach dreimaliger Extraktion mit jeweils 175 g Dichlormethan, Trocknen über Natriumsulfat, Abfiltrieren des Trocknungsmittels und Eindampfen zur Trockene erhält man Thiophenylethanol in einer Ausbeute von 83 %.

### Beispiel 6

Herstellung von Benzoesäure aus Benzol und Chlorcyclohexan (Deprotonierung von Benzol)

Eine Mischung aus 0,2 mol Chlorcyclohexan und 0,2 mol Benzol wird zu einer Suspension von 0,4 mol Lithiumgranalien, 0,21 mol Kalium-tert.-butylat und 35 mg Biphenyl in 300 g THF bei -72°C zugetropft. Nach einem per GC bestimmten Umsatz des Chlorcyclohexans von > 97 % (insgesamt 24 h) wird Kohlendioxid bis zur Sättigung eingeleitet. Die Aufarbeitung geschieht analog zu Beispiel 3; es wird Benzoesäure in einer Ausbeute von 79 % erhalten.

## Patentansprüche

1. Verfahren zur Herstellung von Aryllithiumverbindungen der Formeln (IV) und (VI) durch Umsetzung von Halogenverbindungen (I) mit Lithiummetall zur einer Lithiumverbindung der Formel (II) und weiterer Umsetzung von (II) mit aromatischen Verbindungen der Formeln (III) und/oder (V) unter Deprotonierung und Bildung der Lithiumaromaten (IV) und/oder (VI) (Gleichung I), wobei die Reaktion als Eintopfreaktion durchgeführt wird und die Verbindungen (I), (III) und (V) gleich zeitig oder als Mischung an der Reaktionslösung zudosiert werden und die zuzusetzende Lithiummenge pro Mol umzusetzendes Halogen im Bereich von 1.95 bis 2,5 mol liegt. 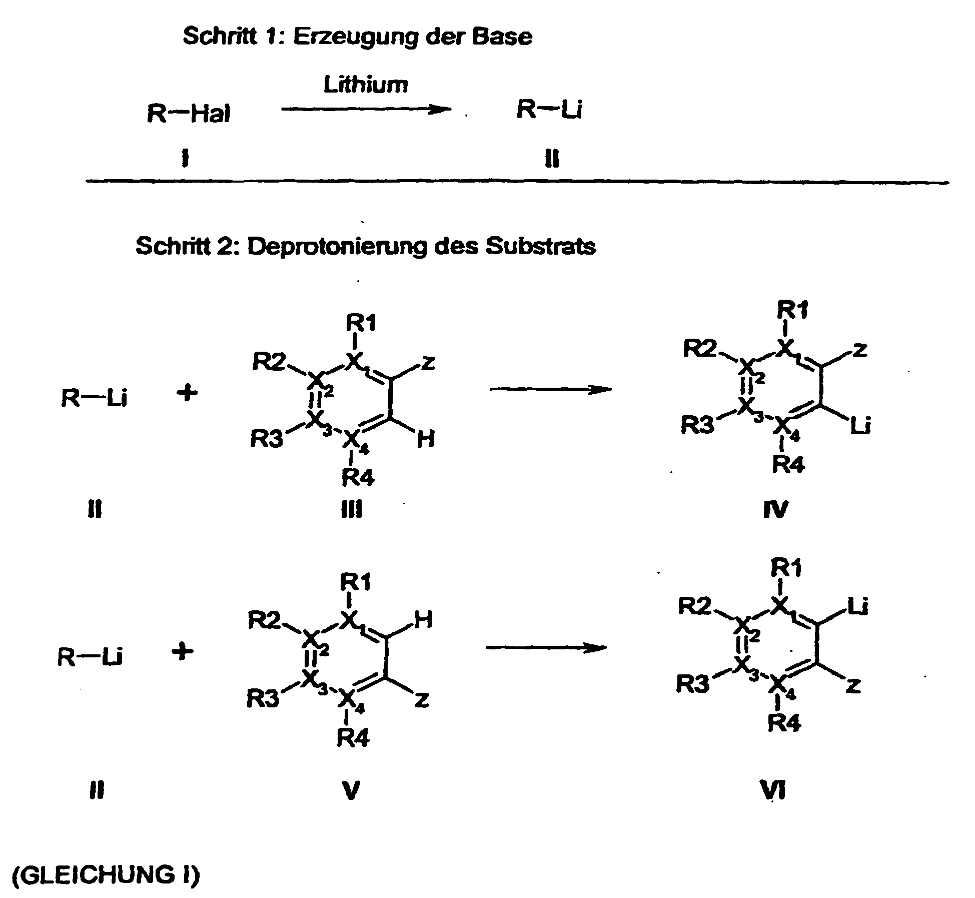 worin R für Methyl, primäre, sekundäre oder tertiäre Alkylreste mit 2 bis 12 C-Atomen, mit einem Rest aus der Gruppe {Phenyl, substituiertes Phenyl, Aryl, Heteroaryl, Alkoxy, Dialkylamino, Alkylthio} substituiertes Alkyl, substituiertes oder unsubstituiertes Cycloalkyl mit 3 bis 8 Atomen steht,
Hal = Fluor, Chlor, Brom oder Iod bedeuten,
X₁₋₄ stehen unabhängig voneinander für Kohlenstoff, die Gruppierung X₁₋₄ R₁₋₄ kann Stickstoff bedeuten, oder zwei benachbarte Reste X₁₋₄ R₁₋₄ können gemeinsam für O, S, NH oder NR' stehen, wobei R' für C₁-C₅-Alkyl, SO₂-Phenyl, SO₂-p-Tolyl oder Benzoyl steht;
die Reste R₁₋₄ und der Rest Z stehen für Substituenten aus der Gruppe {Wasserstoff, Methyl, primäre, sekundäre oder tertiäre, cyclische oder acyclische Alkylreste mit 2 bis 12 C-Atomen, substituierte cyclische oder acyclische Alkylgruppen, Alkoxy, Dialkylamino, Alkylamino, Arylamino, Diarylamino, Phenyl, substituiertes Phenyl, Alkylthio, Diarylphosphino. Dialkylphosphino. Dialkyl- oder Diarylaminocarbonyl, Monoalkyl- oder Monoarylaminocarbonyl, CO₂⁻. Hydroxyalkyl, Alkoxyalkyl. Fluor oder Chlor, CN oder Heteroaryl}, wobei jeweils zwei benachbarte Reste R₁₋₄ zusammen einem aromatischen oder aliphatischen Ring bilden können.

2. Verfahren nach Anspruch 1, **dadurch gekennzeichnet, dass** die Umsetzungen bei Temperaturen im Bereich von -100 bis +25°C durchgeführt werden.

3. Verfahren nach mindestens einem der vorhergehenden Ansprüche, **dadurch gekennzeichnet, dass** die Reaktionen in einem etherischen Lösungsmittel durchgeführt werden.

4. Verfahren nach mindestens einem der vorhergehenden Ansprüche, **dadurch gekennzeichnet dass** bei der Umsetzung des Li-Metalls in der ersten Stufe organische Redoxsysteme zugegeben werden.

5. Verfahren nach mindestens einem der vorhergehenden Ansprüche, **dadurch gekennzeichnet, dass** die Verbindungen der Formeln (III) oder (V) In ortho-Position zum deprotonierenden Wasserstoff Alkoxy, F, Cl, substituiertes Amino, CN. Heteroaryl, Aminoalkyl oder Hydroxyalkyl Reste tragen.

6. Verfahren nach mindestens einem der vorhergehenden Ansprüche, **dadurch gekennzeichnet, dass** die Verbindungen der Formeln (IV) und/oder (VI) anschließend mit einem Elektrophil umgesetzt werden.

7. Verfahren nach mindestens einem der vorhergehenden Anspruche, **dadurch gekennzeichnet, dass** die Umsetzung mit dem Elektrophil entweder nach der Erzeugung der Lithiierten Verbindung (IV) und/oder (V) oder im Eintopfverfahren durch gleichzeitige Zugabe zur Reaktionsmischung erfolgt.

8. Verfahren nach Anspruch 6, **dadurch gekennzeichnet, dass** als Elektrophile Kohlenstoff- Bor- oder Siliciumverbindungen eingesetzt werden.

## Claims

1. A process for preparing aryllithium compounds of the formulae (IV) and (VI) by reacting halogen compounds (I) with lithium metal to form a lithium compound of the formula (II) and reacting (II) further with aromatic compounds of the formulae (III) and/or (V) to deprotonate these and form the lithium aromatics (IV) and/or (VI) (equation I), 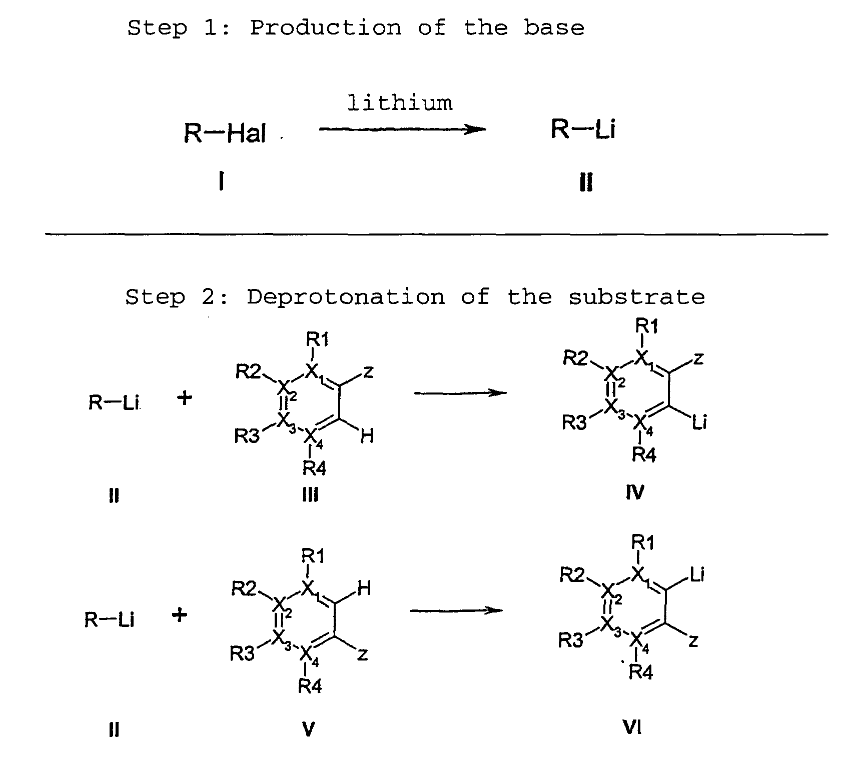 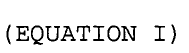 where R is methyl, a primary, secondary or tertiary alkyl radical having from 2 to 12 carbon atoms, alkyl substituted by a radical from the group consisting of {phenyl, substituted phenyl, aryl, heteroaryl, alkoxy, dialkylamino, alkylthio} or substituted or unsubstituted cycloalkyl having from 3 to 8 carbon atoms,
Hal = fluorine, chlorine, bromine or iodine,
X₁₋₄ are, independently of one another, each carbon or the moiety X₁₋₄R₁₋₄ can be nitrogen or two adjacent radicals X₁₋₄R₁₋₄ can together be O, S, NH or NR', where R' is C₁-C₅-alkyl, SO₂-phenyl, SO₂-p-tolyl or benzoyl;
the radicals R₁₋₄ and the radical Z are substituents from the group consisting of {hydrogen, methyl, primary, secondary or tertiary, cyclic or acyclic alkyl radicals having from 2 to 12 carbon atoms, substituted cyclic or acyclic alkyl groups, alkoxy, dialkylamino, alkylamino, arylamino, diarylamino, phenyl, substituted phenyl, alkylthio, diarylphosphino, dialkylphosphino, dialkylaminocarbonyl or diarylaminocarbonyl, monoalkylaminocarbonyl or monoarylaminocarbonyl, CO₂⁻, hydroxyalkyl, alkoxyalkyl, fluorine and chlorine, CN and heteroaryl}, where in each case two adjacent radicals R₁₋₄ can together form an aromatic or aliphatic ring wherein the reaction is carried out as a one-pot reaction and the compounds (I), (III) and/or (V) are added simultaneously or as a mixture to the reaction and the amount of lithium to be added per mole of halogen to be reacted is in the range from 1.95 to 2.5 mol.

2. The process as claimed in claim 1, wherein the reactions are carried out at temperatures in the range from -100 to +25°C.

3. The process as claimed in at least one of the preceding claims, wherein the reactions are carried out in an ether solvent.

4. The process as claimed in at least one of the preceding claims, wherein organic redox systems are added in the reaction of the Li metal in the first stage.

5. The process as claimed in at least one of the preceding claims, wherein the compounds of the formula (III) or (V) bear alkoxy, F, Cl, substituted amino, CN, heteroaryl, aminoalkyl or hydroxyalkyl radicals in the ortho position relative to the deprotonating hydrogen.

6. The process as claimed in at least one of the preceding claims, wherein the compounds of the formulae (IV) and/or (VI) are subsequently reacted with an electrophile.

7. The process as claimed in at least one of the preceding claims, wherein the reaction with the electrophile is carried out either after production of the lithiated compound (IV) and/or (V) or in a one-pot process by simultaneous addition to the reaction mixture.

8. The process as claimed in claim 6, wherein carbon, boron or silicon compounds are used as electrophiles.

## Revendications

1. Procédé pour la préparation de composés aryllithium de formules (IV) et (VI) par mise en réaction de composés halogénés (I) avec du lithium, conduisant à un composé lithié de formule (II) et réaction suivante de (II) avec des composés aromatiques de formule (III) et/ou de formule (V) avec déprotonation et formation des composés aromatiques lithiés (IV) et/ou (VI) (équation I), la réaction étant effectuée en tant que réaction en un seul récipient et les composés (I), (III) et/ou (V) étant ajoutés simultanément ou sous forme de mélange à la solution réactionnelle et la quantité de lithium à ajouter par mole d'halogène à mettre en réaction se situant dans la plage allant de 1,95 à 2,5 moles. 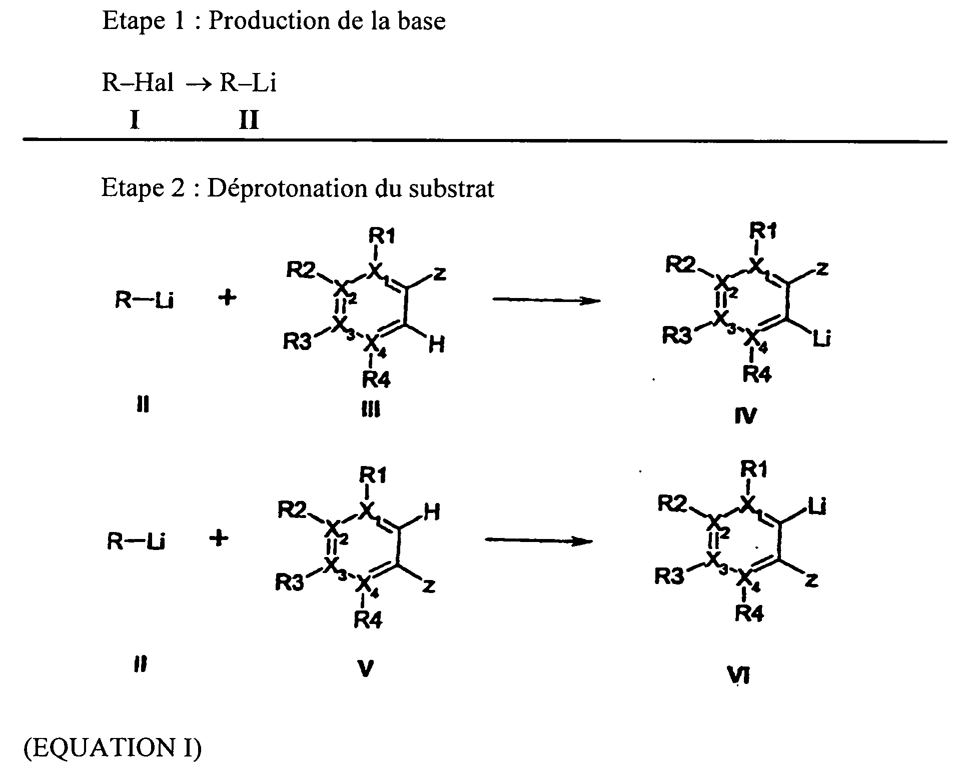 où R représente le groupe méthyle, des radicaux alkyle primaires, secondaires ou tertiaires ayant de 2 à 12 atomes de carbone, un radical alkyle substitué par un groupe choisi dans l'ensemble {phényle, phényle substitué, aryle, hétéroaryle, alcoxy, dialkylamino, alkylthio}, un radical cycloalkyle substitué ou non substitué, ayant de 3 à 8 atomes de carbone,
Hal représente le fluor, le chlore, le brome ou l'iode,
X₁₋₄ représentent, indépendamment les uns des autres, un atome de carbone, le groupement X₁₋₄R₁₋₄ peut représenter un atome d'azote, ou deux radicaux X₁₋₄ R₁₋₄ voisins peuvent représenter ensemble O, S, NH ou NR', R' représentant un groupe alkyle en C₁-C₅, SO₂-phényle, SO₂-p-tolyle ou benzoyle ;
les radicaux R₁₋₄ et le radical Z représentent des substituants choisis dans l'ensemble {atome d'hydrogène, méthyle, radicaux alkyle primaires, secondaires ou tertiaires, cycliques ou acycliques, ayant de 2 à 12 atomes de carbone, groupes alkyle cycliques ou acycliques substitués, alcoxy, dialkylamino, alkylamino, arylamino, diarylamino, phényle, phényle substitué, alkylthio, diarylphosphino, dialkylphosphino, dialkyl- ou diarylaminocarbonyle, monoalkyl- ou monoarylaminocarbonyle, CO₂⁻, hydroxyalkyle, alcoxyalkyle, fluor ou chlore, CN ou hétéroaryle}, chaque fois deux radicaux R₁₋₄ voisins pouvant former ensemble un cycle aliphatique ou aromatique.

2. Procédé selon la revendication 1, **caractérisé en ce que** les réactions sont effectuées à des températures dans la plage de -100 à +25 °C.

3. Procédé selon au moins l'une des revendications précédentes, **caractérisé en ce que** les réactions sont effectuées dans un solvant de type éther.

4. Procédé selon au moins l'une des revendications précédentes, **caractérisé en ce que** lors de la réaction du lithium dans la première étape on ajoute des systèmes redox organiques.

5. Procédé selon au moins l'une des revendications précédentes, **caractérisé en ce que** les composés de formule (III) ou (V) portent des radicaux alcoxy, F, Cl, amino substitué, CN, hétéroaryle, aminoalkyle ou hydroxyalkyle en position ortho par rapport à l'atome d'hydrogène à déprotoner.

6. Procédé selon au moins l'une des revendications précédentes, **caractérisé en ce qu'**on fait ensuite réagir les composés de formule (IV) et/ou de formule (VI) avec un composé électrophile.

7. Procédé selon au moins l'une des revendications précédentes, **caractérisé en ce que** la réaction avec le composé électrophile s'effectue soit après la production du composé lithié (IV) et/ou (V), soit, dans le processus en un seul récipient, par addition simultanée au mélange réactionnel.

8. Procédé selon la revendication 6, **caractérisé en ce qu'**on utilise comme composés électrophiles des composés contenant du carbone, du bore ou du silicium.
